# EUROPEAN PATENT SPECIFICATION

(11) **EP 1 507 570 B1**
(45) Date of publication and mention of the grant of the patent: **25.06.2008**
(21) Application number: 03753079.7
(22) Date of filing: 15.05.2003
(51) Int. Cl.: A61M 25/10

(54) **NON-BUCKLING BALLOON CATHETER**
NICHT-KNICKENDER BALLONKATHETER
CATHETER A BALLONNET INDEFORMABLE

(30) Priority: 16.05.2002 US 381975 P
(43) Date of publication of application: 23.02.2005
(73) Proprietor: Wilson-Cook Medical Inc., Winston-Salem, NC 27115-4191 (US)
(72) Inventor: KENNEDY, Kenneth, C. II, Clemmons, NC 27012 (US)
(74) Representative: Adams, Harvey Vaughan John
(86) International application number: PCT/US2003/015593
(87) International publication number: WO 2003/097152

(56) References cited:
- EP-A- 0 935 973
- EP-A- 1 016 430
- DE-A- 2 653 424
- DE-U- 29 806 831
- FR-A- 2 723 537
- US-A- 4 338 943
- US-A- 5 681 344

## Description

### TECHNICAL FIELD

This invention relates to medical devices, and more particularly to balloon catheters that can be placed within a body lumen and inflated to perform various medical procedures. The invention is especially relevant to balloon catheters with balloons formed of non-elastomeric films or materials, wherein the film that forms the balloon is folded and unfolded during deflation and inflation, respectively, of the balloon.

### BACKGROUND OF THE INVENTION

Balloon catheters are used to perform various medical procedures wherein the balloon is positioned within a body lumen or canal and subsequently inflated. In some of these medical procedures, such as in an angioplasty procedure, the balloon is inflated so as to expand the interior volume of the body canal. In this type of procedure, the balloon is expanded to apply pressure to the interior surface of the body canal to thereby compress any tissue protruding into the canal and thereby enlarge the interior volume thereof. Once the tissue has been compressed, and the body canal widened, the balloon is deflated and removed.

In other types of medical procedures, such as photodynamic therapy (PDT), a balloon catheter is used to align and stabilize the catheter within the body lumen. For example, the balloon catheter may be inflated under low pressure within a body lumen such as the esophagus. A therapeutic fiber optic device is then inserted into the catheter in the vicinity of the balloon. The therapeutic fiber optic device is then used to emit light waves to treat the surrounding tissue. In this procedure, the balloon is used to both align the catheter in the center of the body lumen, and to prevent the catheter from moving during the PDT procedure. However, the tissue to be treated must not be unduly compressed by the expanded balloon. Thus, the balloon is expanded only enough to lightly contact the interior surface of the lumen and align the catheter. Document EP 1016430 discloses a balloon catheter according to the preambles of claims 1 and 14.

As will be explained below, conventional balloon catheters have a number of shortcomings that make them inadequate for many of the above-described procedures, and in particular, for PDT procedures.

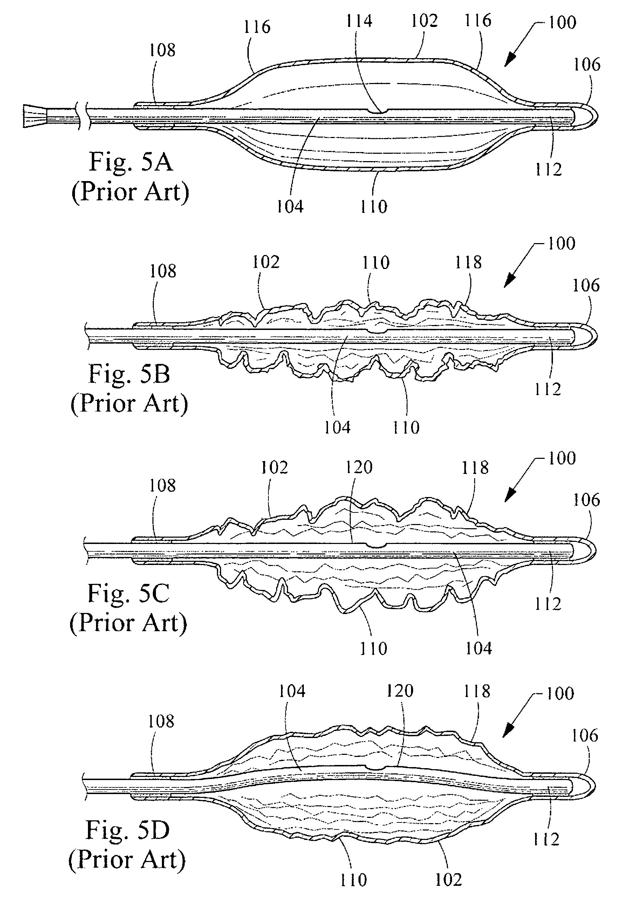

A typical balloon catheter 100 is shown in FIGS. 5A-5D. As best seen in FIG. 5A, a conventional balloon catheter 100 comprises a balloon 102 that is affixed to a catheter 104. The balloon 102 is typically manufactured from a non-elastomeric material (e.g., a semi-rigid or non-compliant material), and includes a distal neck or end 106, a proximal neck or end 108 and a central portion 110. The balloon 102 is affixed to the catheter 104 by inserting the distal end 112 of the catheter 104 into and through the proximal end 108 of the balloon 102. The balloon 102 is then slid over the catheter 104 until the distal end 112 of the catheter 104 is inserted into the distal end 106 of the balloon 102. The distal end 112 of the catheter 104 is then affixed to the distal end 106 of the balloon 102 by an adhesive, ultrasonic welding, or some other method. The proximal end 108 of the balloon 102 is similarly affixed to the outer wall of the catheter 104 so as to anchor and seal the proximal end of the balloon 102.

The catheter 104 includes an aperture 114 for the introduction of air or some other fluid into the interior volume of the balloon 102. Although not shown in the drawings, the proximal end of the catheter 104 is typically attached to a device, such as a syringe, that is manipulated to either inflate or deflate the balloon 102 by injecting a fluid into or withdrawing a fluid from, respectively, the interior volume of the balloon 102.

The conventional balloon catheter 100 has a number of drawbacks for use in many of the above-described procedures, and in particular, for use in PDT procedures. When initially manufactured, the balloon catheter 100 generally assumes a shape and configuration as depicted in FIG. 5A. As can be seen in this drawing, the central portion 110 of the balloon 102 is connected to the distal end 106 and the proximal end 108 by tapered or conical sections 116. The tapered sections 116 provide a transition between the larger diameter of the central portion 110 of the balloon 102 and the outermost portions of the balloon 102 (i.e., the distal end 106 and the proximal end 108) that are connected to the catheter 104.

At the time of packaging by the manufacturer or at the initiation of the medical procedure, the balloon 102 is typically deflated prior to inserting of the balloon catheter 100 into the body canal. Deflation of the balloon 102 is necessary to reduce the overall cross-section or diameter of the device to permit it to pass through an endoscope and/or to navigate and pass through the body's internal canals. FIG. 5B depicts the balloon catheter 100 in the deflated state. As can be seen in this drawing, the balloon 102 is forced to compress in length. This is because the overall length of the material that forms the central portion 110 and the tapered portions 116, as measured along the surface of the balloon 102 in a generally axial direction of the catheter 104 (i.e., from one end of the balloon 102 to the other), is greater than the distance between the distal end 106 and the proximal end 108. As a result of this compression, transverse creases 118 typically form along the surface of the balloon 102.

After the balloon catheter 100 is positioned within the body canal (not shown) at the desired location, inflation of the balloon 102 is initiated as shown in FIG. 5C. As depicted in this drawing, the creases 118 in the surface of the material may prevent the balloon 102 from fully expanding to its normal length (i.e., as shown in FIG. 5A). In other words, the balloon 102 tends to act like a spring under tension. As a result, the portion of the catheter 104 that lies between the distal end 106 and the proximal end 108 of the balloon 102 will be forced into compression, and may begin to bow 120 as a result of these compressive forces.

As inflation of the balloon 102 continues, bowing 120 of the catheter 104 may be increased as shown in FIG. 5D. This is the result of transverse or outward expansion of the central portion 110 of the balloon, which tends to pull the distal end 106 and the proximal end 108 towards each other.

Bowing 120 of the catheter 104 may not be eliminated until a sufficiently high inflation pressure is applied to the balloon 102 (see FIG. 5A). However, some bowing 120 of the catheter 104 may nevertheless remain if the initial deflation of the balloon 102 (see FIG. 5B) resulted in the formation of permanent transverse creases 118. Permanent bowing 120 of the catheter 104 is more likely if the balloon 102 is constructed from a non-elastomeric material.

The formation of transverse creases 118 and the bowing 120 of the catheter 104 can negatively impact the use of the conventional balloon catheter 100 during certain medical procedures. For example, during angioplasty procedures, permanent creases 118 in the surface of the balloon 102 may prevent the complete or uniform compression of the tissue on the interior surface of the body canal against which the balloon 102 is expanded. This may result in a decrease in effectiveness of the angioplasty procedure.

With respect to PDT procedures, any bowing 120 of the catheter 104 can prevent accurate alignment and centering of the catheter 104 within the body lumen or canal to be treated. This is because typical PDT procedures do not allow the expanded balloon 102 to exert excess pressure or heavy contact on the interior surface of the body lumen. Thus, the balloon 102 cannot be inflated with a pressure that is sufficient to eliminate any bowing 120 of the catheter 104. The catheter 104 may consequently not be properly centered in the body lumen. As a result, effective treatment of the body lumen tissue with the therapeutic fiber optic device, which is positioned inside the catheter 104, may be inhibited.

In addition, because the distal end 106 and the proximal end 108 of the balloon 102 are both fixed to the catheter 104 at permanent (i.e., non-moveable) locations, the ability to reduce the diameter of the deflated balloon 102 may be limited, particularly if the balloon 102 is manufactured from a non-elastomeric material. In other words, the central portion 110 of the balloon 102 may not compress tightly about the catheter 104 during deflation because of the creases 118 formed in the material of the balloon 102 (see FIG. 5B). Bunching of the balloon material may likewise limit the deflated diameter or cross-section of the balloon 102. Consequently, the device may be more difficult to maneuver during ingress or egress of the device through the body's canals. In addition, the resulting "wrinkled" surface of the balloon 102 may cause irritation to body canal tissue during ingress or egress of the device and/or prevent the device from passing through the endoscope channel.

What is needed is an improved balloon catheter that overcomes the disadvantages of the conventional devices. In particular, what is needed is a balloon catheter that can be deflated to a minimal diameter for ingress and egress through the body's canals and/or an endoscope channel, that resists the formation of transverse creases in the surface of the balloon during deflation, and that resists bowing of the catheter portion located within the balloon upon inflation.

### SUMMARY OF THE INVENTION

The foregoing problems are solved and a technical advance is achieved by the balloon catheter of the present invention. The balloon catheter includes a rounded or cylindrically shaped balloon that is affixed to a catheter. The balloon includes a distal end, a proximal end and a central portion, and may be formed of a non-elastomeric material. The balloon is attached to the catheter by inserting the distal end of the catheter into and through the proximal end of the balloon until the distal end of the catheter is inserted into a portion of the distal end of the balloon. The proximal end of the balloon is then affixed to the outer wall of the catheter so as to provide an air tight seal between these components.

The distal end of the catheter is not affixed to the distal end of the balloon. Instead, a slip joint is formed between these components. The slip joint allows the distal end of the balloon to axially move or translate with respect to the distal end of the catheter.

Alternatively, the catheter may be terminated so as to not contact the distal end of the balloon, thereby eliminating the slip joint altogether. Or the catheter can be segmented so that separate components are connected to each end of the balloon, but are allowed to move relative to each other.

The above-described configurations allow the overall length of the balloon to change during inflation or deflation, the change in length of the balloon not being impeded by the predetermined length of the catheter. In addition, the above-described configurations prevent the relative axial rigidity of the catheter from generating any axial tensile or compressive forces in the balloon. Consequently, transverse creasing of the central portion of the balloon is eliminated or at least minimized. Moreover, the central portion of the balloon can be collapsed into a smaller diameter or cross-section for ingress or egress of the balloon catheter through the body's canals and/or the endoscope channel.

The slip joint (or the elimination of a continuous catheter connected between both ends of the balloon) also prevents balloon from generating any adverse forces in the catheter during inflation or deflation of the device. In particular, since the distal end of the balloon is not rigidly connected to the distal end of the catheter, any axial contraction or expansion of the balloon will not impart any tensile or compressive forces along the axis of the catheter, and the catheter will not be bowed or stretched as result of the inflation or deflation of the balloon. Consequently, the catheter should remain centered with respect to cross-section of the balloon irrespective of the state of inflation of the balloon.

These and other advantages, as well as the invention itself, will become apparent in the details of construction and operation as more fully described below. Moreover, it should be appreciated that several aspects of the invention can be used with other types of balloon catheters or medical devices.

### BRIEF DESCRIPTION OF SEVERAL VIEWS OF THE DRAWINGS

Several embodiments of the present invention will now be described by way of example with reference to the accompanying drawings, in which:
FIG. 1 is a cross-sectional side view of an illustrative embodiment of a balloon catheter in accordance with the teachings of the present invention;
FIG. 2 is a cross-sectional side view of a second embodiment of a balloon catheter in accordance with the teachings of the present invention;
FIG. 3 is a cross-sectional side view of a third embodiment of a balloon catheter in accordance with the teachings of the present invention;
FIG. 4 is a cross-sectional side view of a fourth embodiment of a balloon catheter in accordance with the teachings of the present invention;
FIGS. 5A-5D depict cross-sectional side views of a conventional balloon catheter in various stages of inflation and deflation;
FIG. 6 is a cross-sectional side view of a fifth embodiment of a balloon catheter in accordance with the teachings of the present invention; and
FIG. 7 is a cross-sectional side view of a sixth embodiment of a balloon catheter in accordance with the teachings of the present invention.

### DETAILED DESCRIPTION OF THE INVENTION

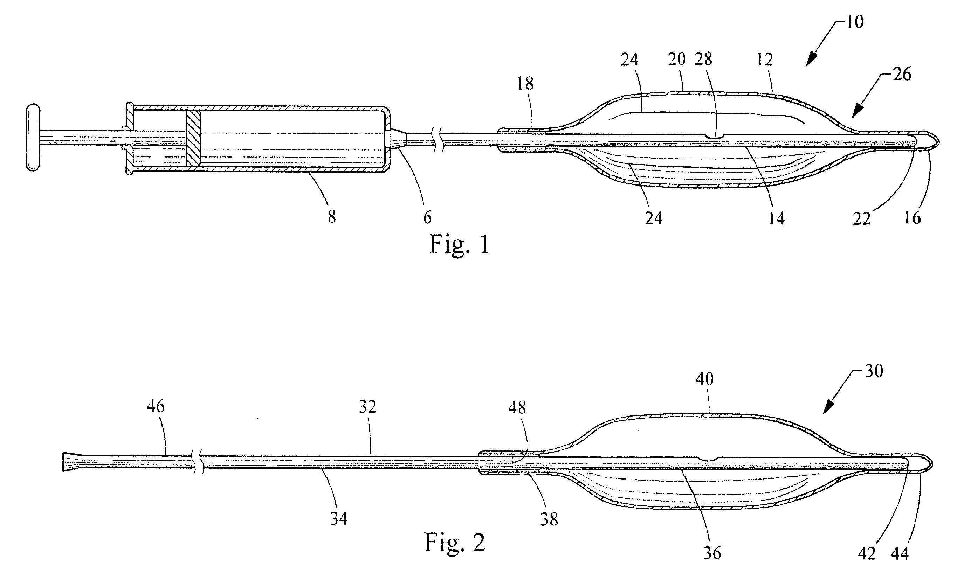

A first embodiment of a balloon catheter 10 of the present invention is depicted in FIG. 1. The balloon catheter 10 includes a rounded, oval, cylindrical, bullet or other appropriately shaped balloon 12 that is affixed to a catheter 14. The balloon 12 is typically manufactured from a non-elastomeric material (e.g., a semi-rigid or non-compliant material), and preferably comprises a translucent, transparent or optically clear film. For example, the balloon 12 could be manufactured from a biocompatible polymer such as polyamide, polyurethane, polyester, polyolefin, polyethylene terephthalate and the like.

The balloon 12, as shown in the drawings, includes a distal end 16, a proximal end 18 and a central portion 20. However, different configurations or designs can also be utilized for the balloon 12. For example, the distal end 16 and the proximal end 18 could both comprise a tubular construction so as to form a neck. The balloon 12 is attached to the catheter 14 by inserting the distal end 22 of the catheter 14 into and through the proximal end 18 of the balloon 12. The balloon 12 is then slid over the catheter 14 until the distal end 22 of the catheter 14 is inserted into a portion of the distal end 16 of the balloon 12. The proximal end 18 of the balloon 12 is then affixed to the outer wall of the catheter 14 by an adhesive, ultrasonic welding, or some other method so as to anchor and seal the proximal end of the balloon 12. In the preferred embodiment shown, the inside diameter of the proximal end 18 is sized to fit tightly or snugly over the catheter 14 so as to improve the integrity of the seal between these two components.

The distal end 22 of the catheter 14 is not affixed to the distal end 16 of the balloon 12. As shown in the drawing, the distal end 22 of the catheter 14 extends partially, but not fully, into the distal end 16 of the balloon 12 so as to form a slip joint 26 between these two components. The slip joint 26 allows the distal end 16 of the balloon 12 to axially move or translate with respect to the distal end 22 of the catheter 14. This configuration allows the overall axial or longitudinal length of balloon 12 to change during inflation or deflation without transferring tensile or compressive forces to the catheter 14. For example, when the balloon 12 is deflated, the balloon 12 tends to elongate in the axial direction as the central portion 20 is drawn inwardly towards the catheter 14, thereby moving the distal end 16 of the balloon 12 distally from or relative to the distal end 22 of the catheter 14. Since the distal end 16 of the balloon 12 is not prevented from moving axially, transverse creasing of the central portion 20 of the balloon 12 during deflation is eliminated or at least minimized. Moreover, the central portion 20 of the balloon 12 can be collapsed into a smaller diameter or cross-section for ingress or egress of the balloon catheter 10 through the body's canals and/or the endoscope channel.

The slip joint 26 also prevents the application of adverse forces on the catheter 14 by the balloon 12 during inflation or deflation of the device. In particular, since the distal end 16 of the balloon 12 is not connected to the distal end 22 of the catheter 14, any axial contraction or expansion of the balloon 12 will not impart any tensile or compressive forces onto the catheter 14. In other words, the catheter 14 will not be bowed or stretched as result of the inflation or deflation of the balloon 12. Consequently, the catheter 14 should remain centered with respect to cross-sectional area of the balloon 12 irrespective of the state of inflation of the balloon 12.

By partially extending the distal end 22 of the catheter 14 into the distal end 16 of the balloon 12, the distal end 22 of the catheter 14 can provide some lateral or transverse support to the distal end 16 of the balloon 12. This lateral support can help to guide the device, and prevent the balloon 12 from folding or collapsing, as the device is being inserted into the body's canals. The length of the distal end 16 of the balloon 12, and the position of the distal end 22 of the catheter 14 therein, should be sufficient to permit these components to freely translate with respect to each other in response to all stages of inflation and deflation of the device.

The distal end 16 of the balloon 12 is sealed so as to enclose the balloon 12. In the preferred embodiment shown, the distal end 16 of the balloon 12 is formed by inserting and sealing a small rod into the neck of the balloon 12. The distal end 16 of the balloon 12 may also be rounded to improve the ingress of the balloon catheter 10 into and through the body's canals and lumens, as well as through the channel of an endoscope. In addition, the inside diameter of the distal end 16 of the balloon 12 is slightly larger than the outside diameter of the distal end 22 of the catheter 14 so as to permit air or fluid to enter or be removed from the interior volume of the balloon 12 by passing through the distal end 22 of the catheter 14. Alternatively, an aperture 28 may be provided in the wall of the catheter 14 at a location proximal to the distal end 22, but within the interior volume of the balloon 12.

The central portion 20 of the balloon 12 may be provided with longitudinally or axially extending pleats or folds 24. These folds 24 provide creases along which the surface of the balloon 12 will fold or pleat when deflated. The folds 24 permit the central portion 20 of the balloon 12 to be collapsed to a minimal cross-sectional area or diameter, and prevent the formation of transverse or lateral creases along the same area.

The proximal end 6 of the catheter 14 is typically connected to an inflation device 8, such as a standard medical syringe. The inflation device 8 is in fluid communication with the interior of the balloon 12 via a lumen extending through the inside of the catheter 14. The catheter 14 may also comprise additional lumens through which contrast fluids or guide wires (not shown) can be passed.

A second embodiment of a balloon catheter 30 of the present invention is depicted in FIG. 2. The balloon catheter of this embodiment 30 is similar to the embodiment of the balloon catheter 10 shown in FIG. 1, but comprises a two-part catheter 32 having a relatively flexible portion 34 and a relatively rigid portion 36. The flexible portion 34 extends from approximately the proximal end 38 of the balloon 40 to the proximal end 46 of the catheter 32. The flexible portion 34 has a similar design and construction as that of the catheter 14 of the first embodiment shown in FIG. 1.

The rigid portion 36 extends from approximately the proximal end 38 of the balloon 40 to the distal end 42 of the catheter 32. In other words, the rigid portion 36 is that portion of the catheter 32 that is disposed within the balloon 40. The rigid portion 36 is less likely to sag under its own weight or the weight of the balloon 40, and may provide increased lateral support to the distal end 44 of the balloon 40. The increased rigidity of the rigid portion 36 of the catheter 32 may be particularly beneficial for use in PDT procedures, where proper centering and alignment of the therapeutic fiber optic device (not shown) within the catheter 32 is critical.

In the embodiment shown, the flexible portion 34 is connected to the rigid portion 36 at a joint 48 that is preferably located within the proximal end 38 of the balloon 40. The proximal end 38 provides reinforcement to the joint 48, as well as improving the integrity of the seal between these components.

With the exception of the two-part catheter 32 described above, the remaining components of the balloon catheter 30 of the second embodiment are the same or similar to the components of the balloon catheter 10 of the first embodiment. A detailed description of these components and their functions will consequently not be repeated here.

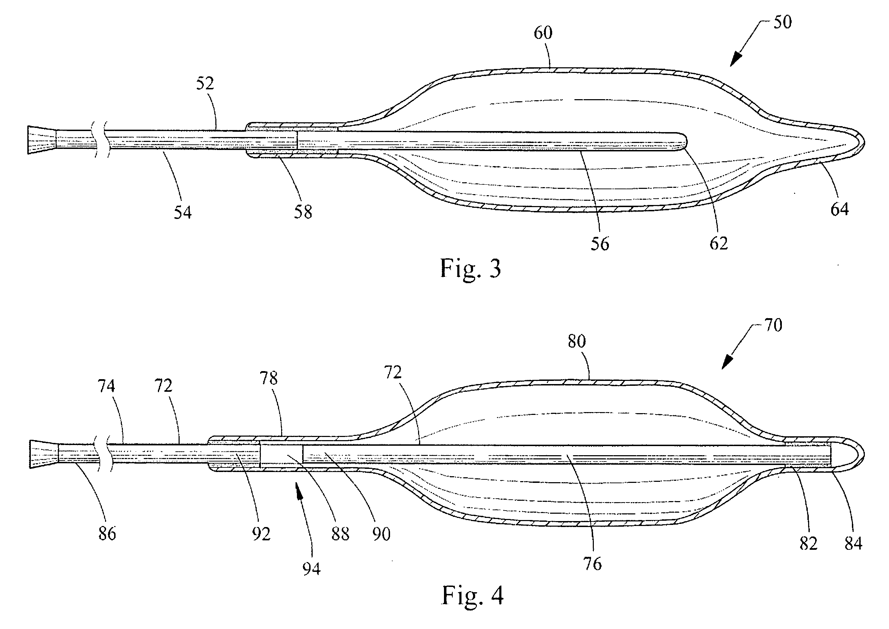

A third embodiment of a balloon catheter 50 of the present invention is depicted in FIG. 3. The balloon catheter 50 of this embodiment is similar to the embodiment of the balloon catheter 30 shown in FIG. 2 in that it also comprises a two-part catheter 52 having a flexible portion 54 and a rigid portion 56. However, the rigid portion 56 does not extend to the distal end 64 of the balloon 60. In other words, the rigid portion 56 only extends from near the proximal end 58 of the balloon 60 to part way into the interior volume of the balloon 60, and the distal end 62 of the rigid portion 56 does not form a slip joint with the distal end 64 of the balloon 60.

With the exception of the two-part catheter 52 described above, and the length of the rigid portion 56 thereof, the remaining components of the balloon catheter 50 of the third embodiment are the same or similar to the components of the balloon catheter 30 of the second embodiment. A detailed description of these components and their functions will consequently not be repeated here.

A fourth embodiment of a balloon catheter 70 of the present invention is depicted in FIG. 4. The balloon catheter of this embodiment 70 is similar to the embodiment of the balloon catheter 10 shown in FIG. 1, but comprises a segmented catheter 72 having a flexible portion 74 and a segmented or spaced apart portion 76. The flexible portion 74 extends from approximately the proximal end 78 of the balloon 80 to the proximal end 86 of the catheter 72. The flexible portion 74 has a similar design and construction as that of the catheter 14 of the first embodiment shown in FIG. 1. The distal end 92 of the flexible portion 74 is affixed to the proximal end 78 of the balloon 80 by adhesive or some other form of bonding. The segmented portion 76 can be either rigid or flexible, and either hollow or solid. In other words, the segmented portion 76 can be a rod-like length of material as opposed to a catheter-like tube since the segmented portion 76 does not necessarily need to carry fluid between the inflation device (not shown) and the balloon 80.

The distal end 82 of the segmented portion 76 is affixed to the distal end 84 of the balloon 80. The segmented portion 76 extends proximally from the distal end 82 and terminates within the proximal end 78 of the balloon 80. The proximal end 90 of the segmented portion 76 is not affixed or bonded to the proximal end 78 of the balloon 80, but is free to move axially within the proximal end 78. In other words, a slip joint 94 is formed between the proximal end 90 of the segmented portion 76 and the proximal end 78 of the balloon 80. A gap 88 is provided between the proximal end 90 of segmented portion 76 and the distal end 92 of the flexible portion 74 within the proximal end 78 of the balloon 80. This gap 88 provides room for the segmented portion 76 to move longitudinally within the proximal end 78 of the balloon 80 as the balloon 80 longitudinally contracts or elongates during inflation and deflation, as well as allowing fluid from the inflation device (not shown) to pass through the distal end 92 of the flexible portion 74 and into the interior of the balloon 80. The proximal end 78 of the balloon 80 also provides lateral support to the proximal end 90 of the segmented portion 76.

This embodiment has the advantage of allowing the balloon 80, and the segmented portion 76 of the catheter 72, to flex near the proximal end 78 of the balloon 80. This may provide increased maneuverability of the balloon catheter 70 during insertion of the device into and through the body's canals.

Of course, it should be appreciated that the segmented portion 76 could terminate short of the proximal end 78 of the balloon 80. In other words, the segmented portion 76 could extend only partially into the interior volume of the balloon 80, thereby eliminating any contact with the proximal end 78 of the balloon 80.

With the exception of the segmented catheter 72 described above, and the location of the slip joint 94 at the proximal end 78 of the balloon 80, the remaining components of the balloon catheter 70 of the fourth embodiment are the same or similar to the components of the balloon catheter 10 of the first embodiment. A detailed description of these components and their functions will consequently not be repeated here.

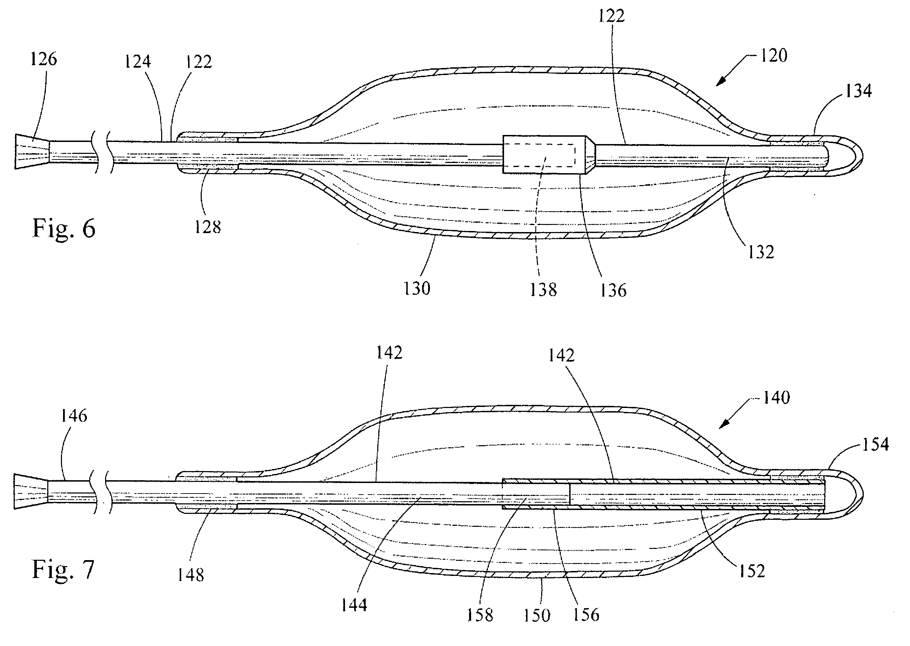

A fifth embodiment of a balloon catheter 120 of the present invention is depicted in FIG. 6. The balloon catheter of this embodiment 120 is similar to the embodiment of the balloon catheter 70 shown in FIG. 4 in that this embodiment comprises a segmented or two-piece catheter 122. However, the proximal portion 124 of the catheter 122 extends from the proximal end 126 of the catheter, through the proximal end 128 of the balloon 130, and into the interior volume of the balloon 130 where it terminates near the mid-section of the balloon 130. The proximal portion 124 of the catheter 122 is affixed to the proximal end 128 of the balloon 130.

The distal portion 132 of the catheter 122 is affixed to the distal end 134 of the balloon 130, and likewise extends into the interior volume of the balloon 130 where it terminates near the mid-section of the balloon 130. The proximal end 136 of the distal portion 132 of the catheter 122 overlaps the distal end 138 of the proximal portion 124 of the catheter 122 in a sliding arrangement. In the embodiment shown, the proximal end 136 of the distal portion 132 of the catheter 122 comprises an expanded tubular portion with an interior diameter that is slightly larger than the exterior diameter of the distal end 138 of the proximal portion 124 of the catheter 122 so as to permit relative axial movement between these two catheter components. This type of connection is often referred to as a male-female type of connection.

A sixth embodiment of a balloon catheter 140 of the present invention is depicted in FIG. 7. The balloon catheter of this embodiment 140 is similar to the embodiment of the balloon catheter 120 shown in FIG. 6 in that this embodiment comprises a segmented or two-piece catheter 142, wherein the proximal portion 144 of the catheter 142 extends from the proximal end 146 of the catheter, through the proximal end 148 of the balloon 150, and into the interior volume of the balloon 150 where it terminates near the mid-section of the balloon 150. The proximal portion 144 of the catheter 142 is affixed to the proximal end 148 of the balloon 150.

The distal portion 152 of the catheter 142 is affixed to the distal end 154 of the balloon 150, and likewise extends into the interior volume of the balloon 150 where it terminates near the mid-section of the balloon 150. The proximal end 156 of the distal portion 152 of the catheter 142 overlaps the distal end 158 of the proximal portion 144 of the catheter 142 in a sliding arrangement. In the embodiment shown, the distal portion 152 of the catheter 142 comprises a uniform tubular cross-section with an interior diameter that is slightly larger than the exterior diameter of the distal end 158 of the proximal portion 144 of the catheter 142 so as to permit relative axial movement between these two catheter components.

In the fifth and sixth embodiments (FIGS. 6 and 7), the overlapping portions of the separate catheter segments provide transverse or lateral stability to the balloon without impeding the axial expansion or contraction of the balloon. This is because the balloon is only fixedly connected to a either one of the catheter portions at single location.

Any other undisclosed or incidental details of the construction or composition of the various elements of the disclosed embodiments of the present invention are not considered to be critical to the achievement of the advantages of the present invention, so long as the elements possess the attributes required to perform as disclosed herein. The selection of these and other details of construction are believed to be well within the ability of one of ordinary skill in the relevant art in view of the present disclosure. Illustrative embodiments of the present invention have been described in considerable detail for the purpose of disclosing practical, operative structures whereby the invention may be practiced advantageously. The designs described herein are intended to be exemplary only. The novel characteristics of the invention may be incorporated in other structural forms without departing from the scope of the invention.

## Claims

1. A balloon catheter (10,30,50,70) comprising:
a catheter (14,32,52,72) having a distal portion and proximal portion; and
a balloon (12,40,60,80) attached to the distal portion of said catheter,
wherein the proximal portion of said catheter is adapted to be connected to an inflation device for inflating or deflating said balloon,
wherein said balloon is fixedly connected to said catheter at only one location so that any axial forces generated in the balloon as a result of an inflation or a deflation of the balloon are not transferred from said balloon to said catheter, and
**characterized in that** said balloon is connected to said catheter at a second location by a slip joint.

2. The balloon catheter according to claim 1 wherein the balloon has a first axial length when inflated and a second axial length when deflated, said first axial length being less than said second axial length.

3. The balloon catheter according to claim 1 or claim 2 wherein said balloon is comprised of a non-compliant or semi-rigid material.

4. The balloon catheter according to claim 3 wherein said balloon comprises axially oriented creases or pleats in the surface thereof to facilitate radial compression of the balloon during deflation.

5. The balloon catheter according to any of the preceding claims wherein the distal portion of the catheter extends through an interior volume of the balloon, and said distal portion of the catheter comprises an aperture disposed within said interior volume of the balloon for permitting the interior volume to be in fluid communication with an inflation device.

6. The balloon catheter according to any of the preceding claims further comprising an inflation device for inflating or deflating said balloon, said inflation device being attached to the proximal portion of said catheter.

7. The balloon catheter according to claim 6 wherein the inflation device comprises a syringe.

8. The balloon catheter according to any of the preceding claims wherein said catheter comprises a flexible portion and a rigid portion, said flexible portion being disposed proximally of said rigid portion.

9. The balloon catheter according to claim 8 wherein said rigid portion is substantially disposed within an interior volume of said balloon.

10. The balloon catheter according to claim 1 wherein said balloon comprises a first end and a second end, said first end being fixedly connected to the catheter at a first location, and said second end being connected to the catheter at the second location by the slip joint.

11. The balloon catheter according to claim 1 wherein said balloon comprises a proximal end and a distal end, said proximal end of said balloon being fixedly connected to the catheter at a first location that is proximal from a distal end of said catheter, and said distal end of said balloon being connected to the distal end of said catheter by the slip joint.

12. The balloon catheter according to claims 1-9 wherein said balloon comprises a proximal end and a distal end, said proximal end of said balloon is fixedly connected to a distal end of a first segment of the catheter, and said distal end of said balloon is connected to a distal end of a second segment of said catheter, said second segment being disconnected from said first segment so as to permit said first and second segments to axially move relative to each other, a proximal end of the second segment of said catheter being connected to the proximal end of said balloon by the slip joint.

13. The balloon catheter according to claim 12 wherein the second segment of said catheter comprises a solid cross-section.

14. A balloon catheter (120,140) comprising:
a catheter (122,142) having a distal portion and proximal portion; and
a balloon (130,150) attached to the distal portion of said catheter;
wherein the proximal portion of said catheter is adapted to be connected to an inflation device for inflating or deflating said balloon,
wherein said balloon is fixedly connected to said catheter at only one location so that any axial forces generated in the balloon as a result of an inflation or a deflation of the balloon are not transferred from said balloon to said catheter, and
wherein said balloon comprises a proximal end and a distal end, said proximal end of said balloon is fixedly connected to a proximal segment of the catheter and **characterized in that** said distal end of said balloon is connected to a distal segment of said catheter, said proximal segment having a distal end portion that is disposed within an interior volume of the balloon, said distal segment having a proximal end portion that is disposed within the interior volume of the balloon, wherein the distal end portion of the proximal segment is in sliding engagement with the proximal end portion of the distal segment so as to permit said proximal and distal segments to axially move relative to each other.

15. The balloon catheter according to claim 14 wherein the distal end portion of the proximal segment and the proximal end portion of the distal segment each comprise a tubular cross-section, and further wherein one of the distal end portion of the proximal segment and the proximal end portion of the distal segment has an exterior diameter that is sized to fit within an interior diameter of the other in a male-female connection.

## Patentansprüche

1. Ballonkatheter (10, 30, 50, 70), umfassend:
einen Katheter (14, 32, 52, 72) mit einem distalen Abschnitt und einem proximalen Abschnitt; und einen am distalen Abschnitt des Katheters befestigten Ballon (12, 40, 60, 80), worin der proximale Abschnitt des Katheters zum Aufblasen oder Entleeren des Ballons mit einer Aufblasvorrichtung verbunden werden kann, worin der Ballon nur an einer Stelle fest mit dem Katheter verbunden ist, so dass im Ballon durch Aufblasen oder Entleeren des Ballons entstehende Axialkräfte nicht vom Ballon auf den Katheter übertragen werden, und **dadurch gekennzeichnet, dass** der Ballon an einer zweiten Stelle durch eine Gleitverbindung mit dem Katheter verbunden ist.

2. Ballonkatheter nach Anspruch 1, worin der Ballon beim Aufblasen eine erste axiale Länge und beim Entleeren eine zweite axiale Länge aufweist, wobei die erste axiale Länge kleiner ist als die zweite axiale Länge.

3. Ballonkatheter nach Anspruch 1 oder Anspruch 2, worin der Ballon aus einem unnachgiebigen oder halbstarren Material besteht.

4. Ballonkatheter nach Anspruch 3, worin der Ballon axial orientierte Falten oder Krausen in seiner Oberfläche umfasst, um radiale Kompression des Ballons beim Entleeren zu erleichtern.

5. Ballonkatheter nach einem der vorhergehenden Ansprüche, worin der distale Abschnitt des Katheters sich durch ein Innenvolumen des Ballons erstreckt, und wobei der distale Abschnitt des Katheters eine Öffnung umfasst, die im Innenvolumen des Ballons angeordnet ist, damit das Innenvolumen in Fluidverbindung mit einer Aufblasvorrichtung stehen kann.

6. Ballonkatheter nach einem der vorhergehenden Ansprüche, umfassend eine Aufblasvorrichtung zum Aufblasen oder Entleeren des Ballons, wobei die Aufblasvorrichtung am proximalen Abschnitt des Katheters befestigt ist.

7. Ballonkatheter nach Anspruch 6, worin die Aufblasvorrichtung eine Spritze umfasst.

8. Ballonkatheter nach einem der vorhergehenden Ansprüche, worin der Katheter einen flexiblen Abschnitt und einen starren Abschnitt umfasst, wobei der flexible Abschnitt proximal zum starren Abschnitt angeordnet ist.

9. Ballonkatheter nach Anspruch 8, worin der starre Abschnitt im Wesentlichen in einem Innenvolumen des Ballons angeordnet ist.

10. Ballonkatheter nach Anspruch 1, worin der Ballon ein erstes und ein zweites Ende umfasst, wobei das erste Ende an einer ersten Stelle fest mit dem Katheter verbunden ist und wobei das zweite Ende an der zweiten Stelle durch die Gleitverbindung mit dem Katheter verbunden ist.

11. Ballonkatheter nach Anspruch 1, worin der Ballon ein proximales Ende und ein distales Ende umfasst, wobei das proximale Ende des Ballons an einer ersten Stelle, die proximal von einem distalen Ende des Katheters liegt, fest mit dem Katheter verbunden ist und wobei das distale Ende des Ballons durch die Gleitverbindung mit dem distalen Ende des Katheters verbunden ist.

12. Ballonkatheter nach Anspruch 1-9, worin der Ballon ein proximales Ende und ein distales Ende umfasst, wobei das proximale Ende des Ballons fest mit einem distalen Ende eines ersten Segments des Katheters verbunden ist, und wobei das distale Ende des Ballons mit einem distalen Ende eines zweiten Segments des Katheters verbunden ist, wobei das zweite Segment vom ersten Segment gelöst wird, damit sich die ersten und zweiten Segmente axial relativ zueinander bewegen können, wobei ein proximales Ende des zweiten Segments des Katheters mit dem proximalen Ende des Ballons durch die Gleitverbindung verbunden ist.

13. Ballonkatheter nach Anspruch 12, worin das zweite Segment des Katheters einen soliden Querschnitt umfasst.

14. Ballonkatheter (120, 140), umfassend:
einen Katheter (122, 142) mit einem distalen Abschnitt und einem proximalen Abschnitt; und einen am distalen Abschnitt des Katheters befestigten Ballon (130, 150), worin der proximale Abschnitt des Katheters zum Aufblasen oder Entleeren des Ballons mit einer Aufblasvorrichtung verbunden werden kann, worin der Ballon nur an einer Stelle fest mit dem Katheter verbunden ist, so dass im Ballon durch Aufblasen oder Entleeren des Ballons entstehende Axialkräfte nicht vom Ballon auf den Katheter übertragen werden, und worin der Ballon ein proximales Ende und ein distales Ende umfasst, wobei das proximale Ende des Ballons fest mit einem proximalen Segment des Katheters verbunden ist und **dadurch gekennzeichnet, dass** das distale Ende des Ballons mit einem distalen Segment des Katheters verbunden ist, wobei das proximale Segment einen distalen Endabschnitt aufweist, der in einem Innenvolumen des Ballons angeordnet ist, wobei das distale Segment einen proximalen Endabschnitt aufweist, der im Innenvolumen des Ballons angeordnet ist, worin der distale Endabschnitt des proximalen Segments mit dem proximalen Endabschnitt des distalen Segments in Gleiteingriff steht, damit sich die proximalen und distalen Segmente axial relativ zueinander bewegen können.

15. Ballonkatheter nach Anspruch 14, worin der distale Endabschnitt des proximalen Segments und der proximale Endabschnitt des distalen Segments jeweils einen röhrenförmigen Querschnitt umfassen, und worin ferner einer des distalen Endabschnitts des proximalen Segments und des proximalen Endabschnitts des distalen Segments einen Außendurchmesser aufweist, der eine solche Größe aufweist, dass er als Steckverbindung in einen Innendurchmesser des anderen passt.

## Revendications

1. Cathéter à ballonnet (10, 30, 50, 70) comprenant:
un cathéter (14, 32, 52, 72) ayant une portion distale et une portion proximale ; et
un ballonnet (12, 40, 60, 80) attaché à la portion distale dudit cathéter ;
la portion proximale dudit cathéter étant prévue pour être connectée à un dispositif de gonflage pour gonfler ou dégonfler ledit ballonnet,
ledit ballonnet étant connecté fixement audit cathéter en seulement un endroit de sorte que toutes les forces axiales produites dans le ballonnet suite au gonflage ou au dégonflage du ballonnet ne soient pas transmises dudit ballonnet audit cathéter, et
**caractérisé en ce que**
ledit ballonnet est connecté audit cathéter en un deuxième endroit par un joint à glissière.

2. Cathéter à ballonnet selon la revendication 1, dans lequel le ballonnet a une première longueur axiale lorsqu'il est gonflé et une deuxième longueur axiale lorsqu'il est dégonflé, ladite première longueur axiale étant inférieure à ladite deuxième longueur axiale.

3. Cathéter à ballonnet selon la revendication 1 ou 2, dans lequel ledit ballonnet se compose d'un matériau non flexible ou semi-rigide.

4. Cathéter à ballonnet selon la revendication 3, dans lequel ledit ballonnet comprend des plissages ou des plis orientés axialement dans sa surface pour faciliter la compression radiale du ballonnet lorsqu'il est dégonflé.

5. Cathéter à ballonnet selon l'une quelconque des revendications précédentes, dans lequel la portion distale du cathéter s'étend à travers un volume intérieur du ballonnet, et ladite portion distale du cathéter comprend une ouverture prévu à l'intérieur dudit volume intérieur du ballonnet pour permettre audit volume intérieur d'être en communication fluidique avec un dispositif de gonflage.

6. Cathéter à ballonnet selon l'une quelconque des revendications précédentes, comprenant en outre un dispositif de gonflage destiné à gonfler ou dégonfler ledit ballonnet, ledit dispositif de gonflage étant attaché à la portion proximale dudit cathéter.

7. Cathéter à ballonnet selon la revendication 6, dans lequel le dispositif de gonflage comprend une seringue.

8. Cathéter à ballonnet selon l'une quelconque des revendications précédentes, dans lequel ledit cathéter comprend une portion flexible et une portion rigide, ladite portion flexible étant disposée à proximité de ladite portion rigide.

9. Cathéter à ballonnet selon la revendication 8, dans lequel ladite portion rigide est disposée substantiellement dans un volume intérieur dudit ballonnet.

10. Cathéter à ballonnet selon la revendication 1, dans lequel ledit ballonnet comprend une première extrémité et une deuxième extrémité, ladite première extrémité étant connectée fixement au cathéter en un premier endroit, et ladite deuxième extrémité étant connectée au cathéter au niveau du deuxième endroit par le joint à glissière.

11. Cathéter à ballonnet selon la revendication 1, dans lequel ledit ballonnet comprend une extrémité proximale et une extrémité distale, ladite extrémité proximale dudit ballonnet étant connectée fixement au cathéter en un premier endroit qui est à proximité d'une extrémité distale dudit cathéter, et ladite extrémité distale dudit ballonnet étant connectée à l'extrémité distale dudit cathéter par le joint à glissière.

12. Cathéter à ballonnet selon l'une quelconque des revendications 1 à 9, dans lequel ledit ballonnet comprend une extrémité proximale et une extrémité distale, ladite extrémité proximale dudit ballonnet étant connectée fixement à une extrémité distale d'un premier segment du cathéter, et ladite extrémité distale dudit ballonnet étant connectée à une extrémité distale d'un deuxième segment dudit cathéter, ledit deuxième segment étant déconnecté dudit premier segment de manière à permettre auxdits premier et deuxième segments de se déplacer axialement l'un par rapport à l'autre, une extrémité proximale du deuxième segment dudit cathéter étant connectée à l'extrémité proximale dudit ballonnet par le joint à glissière.

13. Cathéter à ballonnet selon la revendication 12, dans lequel le deuxième segment dudit cathéter comprend une section transversale pleine.

14. Cathéter à ballonnet (120, 140) comprenant :
un cathéter (122, 142) ayant une portion distale et une portion proximale ; et
un ballonnet (130, 150) attaché à la portion distale dudit cathéter ;
la portion proximale dudit cathéter étant prévue pour être connectée à un dispositif de gonflage pour gonfler ou dégonfler ledit ballonnet,
ledit ballonnet étant connecté fixement audit cathéter en seulement un endroit de sorte que toutes les forces axiales produites dans le ballonnet suite au gonflage ou au dégonflage du ballonnet ne soient pas transmises dudit ballonnet audit cathéter, et
ledit ballonnet comprenant une extrémité proximale et une extrémité distale, ladite extrémité proximale dudit ballonnet étant connectée fixement à un segment proximal du cathéter et **caractérisé en ce que** ladite extrémité distale dudit ballonnet est connectée à un segment distal dudit cathéter, ledit segment proximal ayant une portion d'extrémité distale qui est disposée dans un volume intérieur du ballonnet, ledit segment distal ayant une portion d'extrémité proximale qui est disposée dans le volume intérieur du ballonnet, la portion d'extrémité distale du segment proximal étant en engagement coulissant avec la portion d'extrémité proximale du segment distal de manière à permettre auxdits segments proximal et distal de se déplacer axialement l'un par rapport à l'autre.

15. Cathéter à ballonnet selon la revendication 14, dans lequel la portion d'extrémité distale du segment proximal et la portion d'extrémité proximale du segment distal comprennent chacune une section transversale tubulaire et dans lequel l'une de la portion d'extrémité distale du segment proximal et de la portion d'extrémité proximale du segment distal a en outre un diamètre extérieur qui est dimensionné de manière à s'adapter dans un diamètre intérieur de l'autre, dans une connexion mâle-femelle.
